# NEUE EUROPÄISCHE PATENTSCHRIFT

(11) **EP 0 544 869 B2**
(45) Veröffentlichungstag und Bekanntmachung des Hinweises auf die Entscheidung über den Einspruch: **05.09.2001**
(45) Hinweis auf die Patenterteilung: 10.01.1996
(21) Anmeldenummer: 92912568.0
(22) Anmeldetag: 15.06.1992
(51) Int. Cl.: G01N 33/543, G01N 33/537, G01N 33/564, G01N 33/567, G01N 33/577

(54) **IMMUNOLOGISCHES BESTIMMUNGSVERFAHREN ZUR BESTIMMUNG VON ANTIKÖRPERN IN BIOLOGISCHEN FLÜSSIGKEITEN SOWIE KIT ZUR DURCHFÜHRUNG DES VERFAHRENS**
IMMUNOLOGICAL TEST FOR THE PRESENCE OF ANTIBODIES IN BIOLOGICAL FLUIDS, AND A KIT FOR CARRYING OUT THE TEST
PROCEDE IMMUNOLOGIQUE DE DEPISTAGE D'ANTICORPS DANS DES LIQUIDES BIOLOGIQUES ET KIT DE MISE EN OEUVRE DU PROCEDE

(30) Priorität: 20.06.1991 DE 4120412
(43) Veröffentlichungstag der Anmeldung: 09.06.1993
(73) Patentinhaber: B.R.A.H.M.S Diagnostica GmbH, D-12099 Berlin (DE)
(72) Erfinder: BERGMANN, Andreas, D-1000 Berlin 47 (DE)
(74) Vertreter: Andrae, Steffen, Dr.
(86) Internationale Anmeldenummer: EP9201348
(87) Internationale Veröffentlichungsnummer: WO9300587

(56) Entgegenhaltungen:
- CHEMICAL ABSTRACTS, Band 114, Nr. 11, 18 März 1991, Columbus, OH (US); D.L. KENDLER et al., Nr. 99491t, p. 523;
- AKTUELLE ENDOKRINOLOGIE UND STOFFWECHSEL, Band 10, November 1989, Stuttgart (DE); P.-M. SCHUMM-DRAEGER et al., Seiten 90-102
- AUTOIMMUNITY, Band 7, Nr. 1, 1990, Chur-London-New York; J. FURMANIAK et al., Seiten 63-80
- AUTOIMMUNITY, Band 7, Nr. 2-3, 1990, Chur-London-New York; M. LUDGATE et al., Seiten 201-211
- CLINICAL CHEMISTRY, vol. 35, no. 9, September 1989, Winston (US); K. BEEVER et al.; pp. 1949-1954;
- BIOLOGICAL ABSTRACTS, vol. 76, no. 7, 1983, Philadelphia, PA (US); S. MARIOTTI et al.; abstract no. 48619;
- ENDOCRINOLOGY, vol. 125, no. 3, September 1989, Baltimore (US); J. RUF et al.; pp. 1211-1218;

## Beschreibung

Die Erfindung betrifft ein immunologisches Bestimmungsverfahren zur Bestimmung von Autoantikörpern, deren Nachweis die Diagnose einer Autoimmunerkrankung ermöglicht, und zwar von Autoantikörpern gegen TPO.

Immunologische Bestimmungsverfahren spielen in zahlreichen Varianten eine sehr wesentliche Rolle in der medizinischen Diagnostik. Neben derartigen Bestimmungsverfahren, die auf die qualitative und/oder quantitative Bestimmung von Antigenen oder Haptenen, z.B. Hormonen, abzielen, gibt es auch zahlreiche derartige Bestimmungsverfahren zur Bestimmung von Antikörpem in biologischen Flüssigkeiten, insbesondere humanen Seren.

Antikörper sind Eiweißkörper, die als Immunglobuline (Ig) bezeichnet werden und die vom Körper als Reaktion auf ein Antigen gebildet werden. Da Antigene normalerweise zahlreiche antigene Determinanten aufweisen, sind Antikörper polyklonaler Natur, stellen also eine Population von Eiweißkörpem mit unterschiedlichen Bindungseigenschaften gegenüber dem Antigen, gegen das sie gerichtet sind, dar. Sie werden normalerweise gegen körperfremde Antigene gebildet, um den Körper gegen Substanzen zu schützen die entsprechende antigene Determinanten aufweisen. Erkennt das Immunsystem des Körpers fälschlich bestimmte körpereigene Zellen oder Zellstrukturen als fremd, können aber auch Antikörper gegen Antigendeterminanten körpereigener Elemente gebildet werden. Derartige körpereigene Elemente werden dann als Autoantigene bezeichnet, und die gegen sie gebildeten Antikörper als Autoantikörper.

Bekannte Bestimmungsverfahren für Antikörper verwirklichen in der einen oder anderen Form verschiedene Grundprinzipien, von denen zwei beispielsweise in der US-PS B1 3 654 090 in Spalte 3, oben, schematisch dargestellt sind. Gemäß einer Variante, die dem klassischen Radioimmunoassay (RIA) entspricht, wird ein Unterschuß eines immobilisierten Antigens verwendet, und der zu untersuchenden Probe wird eine markierte Form des zu bestimmenden Antikörpers in einer bekannten Menge zugesetzt. Aus dem Grad der Bindung des markierten Antikörpers an das immobilisierte Antigen kann auf die Anwesenheit bzw. Konzentration des gesuchten Antikörpers zurückgeschlossen werden. Für diesen nach dem Konkurrenzprinzip arbeitenden Test wird das Antigen in hochreiner Form benötigt.

Gemäß einem zweiten Verfahrensprinzip wird so vorgegangen, daß eine bekannte Menge des zu bestimmenden Antikörpers oder eines geeigneten Derivats davon an einem festen Träger immobilisiert wird, und man läßt dann den in der zu untersuchenden Probe vorhandenen Antikörper und den immobilisierten Antikörper um ein dem Reaktionssystem zugesetztes markiertes Antigen konkurrieren. Die Anwesenheit bzw. Menge des zu bestimmenden Antikörpers ergibt sich aus der Verminderung der Bindung des markierten Antigens an den immobilisierten Antikörper und damit an die feste Phase.

Bei der zuletzt genannten Art der Bestimmung wird eine markierte Form des zugehörigen hochreinen Antigens benötigt, und der zu bestimmende Antikörper und der immobilisierte Antikörper müssen in solchen Mengen vorliegen, daß es, gegebenenfalls unter Berücksichtigung nicht völlig identischer Affinitäten zu dem markierten Antigen, zu einer wirksamen Konkurrenz zwischen dem immobilisierten Antikörper und dem Antikörper in der zu untersuchenden Probe kommen kann.

Gemäß einem weiteren Verfahrensprinzip wird in analoger Vorgehensweise zu dem für die Antigenbestimmung gut bekannten Sandwich-Test zuerst ein Überschuß eines Antigens, üblicherweise in immobilisierter Form, vorgelegt, durch das die Gesamtmenge des zu bestimmenden Antikörpers gebunden wird, und durch eine nachfolgende zweite immunologische Umsetzung mit einem zweiten markierten "Antigen" gegen den im ersten Schritt gebundenen Antikörper wird dieser unter Ausbildung eines sandwichartigen Immunkomplexes markiert. Das zweite "Antigen" ist dabei häufig ein anti-Antikörper (Doppelantikörperverfahren), oder es wird zur Markierung das markierte sog. Protein A eingesetzt, ein aus Bakterien gewonnenes Protein, das unspezifisch an zahlreiche IgG-Antikörper bindet. Bei diesem Verfahren werden solche Mengen an Antigen benötigt, daß die Bindungskapazität zur Bindung aller in der Probe vorhandenen Antikörper ausreicht. Muß mit größeren Mengen der zu bestimmenden Antikörper gerechnet werden, müssen die Proben daher in der Regel mehrfach verdünnt werden, bevor sie in den Test eingesetzt werden können. Das gilt insbesondere für die aus praktischen Gründen häufig zu bevorzugende "coated tube"-Technik und Mikrotiterplatten-Technik, bei der ein mit Antigen beschichtetes Teströhrchen nur eine Bindungskapazität von ca. 1-2 µg humanen Antikörpem aufweist. Aus DE 3 907 651 ist ein Verfahren zur Bestimmung von Antikörpern, insbesondere gegen das Hepatitis B-Core Antigen, bekannt, bei dem man mit zwei Antikörpern, von denen einer an eine spezifisch bindefähige Substanz für seine Immobilisierung gebunden ist und der andere markiert ist, und einem Antigen arbeitet und das als "kompetitiver Sandwich assay" bezeichnet werden kann, weit die nach zuweisenden Antikörper mit den beiden als Reagenzien eingesetzten Antikörpern und Bindungsstellen des Antigens kompetieren können und dadurch den Sandwich aufbau stören.

Bei der Bestimmung von Antikörpem gegen körperfremde Antigene lassen sich die Funktionsvoraussetzungen fur die verschiedenen Tests häufig ohne größere Schwierigkeiten erfüllen. Die Antikörperkonzentrationen gegen körperfremde Antigene sind im Normalfalle relativ niedrig, und die zugehörigen Antigene oder auch Haptene können häufig in ausreichenden Mengen auf chemischem oder biotechnologischem Wege synthetisiert oder aus natürlichem Material isoliert und angereichert werden.

Beabsichtigt man jedoch, nach einem der geschilderten Verfahrensprinzipien Autoantikörperzu bestimmen, so stößt man auf eine Reihe von teilweise erheblichen Schwierigkeiten, und zwar sowohl aufgrund der auftretenden Autoantikörperkonzentrationen als auch aufgrund der Natur der Autoantigene.

Die Bestimmung von Autoantikörpem ist für den Nachweis des Vorliegens einer Autoimmunerkrankung sehr wichtig, insbesondere um die beobachteten Krankheitserscheinungen richtig zu interpretieren und evtl. schädliche Falschbehandlungen zu vermeiden. Bekannte Autoimmunerkrankungen, die teilweise außerordentlich schwerer Natur sind, sind beispielsweise die rheumatoide Arthritis, der Diabetes mellitus Typ 1, die Myasthenia gravis sowie einige mit der Schilddrüse assoziierte Autoimmunerkrankungen, wie die Basedow-Hyperthyreose (auch Graves-Krankheit genannt), die Myxödemanämie und die Hashimoto Thyreoiditis. Als Autoantigene wirken im Falle der Schilddrüsen-Autoimmunerkrankungen je nach Art der Krankheit das Thyreoglobulin, der TSH-Rezeptor und/oder die Schilddrüsenperoxidase (TPO), wobei letztere nach jüngeren Erkenntnissen mit dem sogenannten mikrosomalen Antigen identisch ist. Die vorliegende Erfindung betrifft die Bestimmung von Schilddrüsenautoantikörpern, und zwar von Antikörpern gegen hTPO.

Zusammenfassungen des derzeitigen Erkenntnisstandes auf dem Gebiet der Schilddrüsen-Autoimmunerkrankungen finden sich in der wissenschaftlichen Literatur beispielsweise in dem Artikel von Marian Ludgate und Gilbert Vassart in: Autoimmunity, 1990, Band 7, Seiten 201-211; ferner in dem Review von Jadwiga Furmaniak und Bernard Rees Smith in: Autoimmunity, 1990, Band 7, Seiten 63-80; sowie in dem Artikel von P.-M. Schumm-Drager, H.J.C. Wenisch in: Akt. Endokr. Stoffw. 10 (1989), Seiten 9-102 (Sonderheft), wo ein Überblick über die Methoden zum Nachweis von Schilddrüsenautoantikörpern gegeben wird.

Die immundiagnostische Bestimmung von Schilddrüsen-Autoantikörpern bzw. Autoantikörpem generell unter sinngemäßer Anwendung eines der eingangs genannten Bestimmungstypen stößt auf die grundsätzliche Schwierigkeit, daß die Autoantikörper sehr häufig gegen Autoantigene gerichtet sind, die in der Zellmembran verankert sind und in der für die übliche Verfahrensdurchführung erforderlichen hohen Reinheit und Menge nur schwer erhältlich sind. Im Falle der menschlichen Schilddrüsenperoxidase (hTPO), eines Enzyms, das als Autoantigen für die Hashimoto Thyreoiditis verantwortlich ist, handelt es sich beispielsweise um ein glycosyliertes Hämoprotein, das an die Schilddrüsenmembranen gebunden ist. Seine antigenen Eigenschaften, einschließlich der vorhandenen Typen von Epitopen auf seiner Oberfläche, sind beschrieben in dem Artikel von P. Carayon et al. in: Endocrinology, Vol. 125, No.3, S. 1211 bis 1218. Um für die immundiagnostischen Bestimmungsverfahren nach den bekannten Verfahrensprinzipien diese Schilddrüsenperoxidase in ausreichender Reinheit und Menge als Antigen zur Verfügung zu haben, muß die Schilddrüsenperoxidase auf proteolytischem Wege oder mit Hilfe von Detergenzien aus der Membran herausgelöst und über Immunadsorbenzien oder mittels konventioneller Chromatographiemethoden an unterschiedlichen Trennphasen gereinigt werden, z.B. mittels Gelfiltration, lonenaustauschchromatographie, Chromatographie über hydrophobe Interaktionen, Chromatographie über aromatische Interaktionen, Adsorptionschromatographie und Chromatographie über Concanavalin A. Diese Verfahren sind aufwendig und mit dem Risiko ungewollter Veränderungen des zu isolierenden Enzyms sowie starkem Materialverlust verbunden. Hochgereinigte native Schilddrüsenperoxidase (TPO) ist daher nur in geringen Mengen und zu hohen Preisen verfügbar. Als Altemative zur Isolierung der Schilddrüsenperoxidase aus Schilddrüsen wurden daher auch schon Versuche unternommen bzw. Verfahren entwickelt, nach denen TPO auf gentechnologischem Wege erzeugbar ist. Auch die so gewonnene TPO ist jedoch nur in begrenzten Mengen und zu hohen Preisen verfügbar, und die Identität des gentechnologisch gewonnenen Materials mit der natürlichen Schilddrüsenperoxidase, insbesondere was die antigenen Eigenschaften angeht, ist nicht in jedem Falle garantiert.

Eine weitere Schwierigkeit ergibt sich ferner daraus, daß die antigenen Eigenschaften der TPO sehr stark durch chemische Einwirkungen beeinträchtigt werden können, insbesondere wenn dadurch die dreidimensionale Struktur verändert wird und/oder die Disulfidbrücken geöffnet werden (vgl. den genannten Artikel von P. Carayon). Um TPO jedoch als markiertes Antigen in dem klassischen Verfahren zur Antikörperbestimmung einsetzen zu können, muß an das TPO ein Markierungsanteil chemisch gebunden werden. Zusätzlich zu der Schwierigkeit der Gewinnung von reinem TPO besteht auf dieser Stufe das Risiko, daß durch die mit der Markierung verbundenen Umsetzungen die antigenen Eigenschaften der TPO so beeinflußt werden, daß sie nicht mehr der nativen TPO entspricht und daher auch als Antigen zum Nachweis von Autoantikörpern nur noch bedingt geeignet ist. Beispielsweise können die durch die Isolierung und/oder Markierung der TPO bewirkten Veränderungen dazu führen, daß nur noch ein Teil der polyklonalen TPO-Autoantikörper mit einer derartigen markierten TPO reagiert.

Um die mit der Isolierung und Markierung von TPO verbundenen Probleme wenigstens teilweise zu umgehen, wurde als Abwandlung des eingangs geschilderten Sandwich-Tests zur Antikörperbestimmung auch schon ein Test entwickelt, bei dem als immobilisertes Antigen ein nicht hochgereinigt, sondern roh eingesetztes TPO verwendet wird. Bei diesem Test besteht jedoch die Gefahr, daß in dem immobilisierten rohen TPO noch andere Substanzen mit antigenen Eigenschaften vorhanden sind, die zu einer Immobilisierung anderer als der gesuchten Antikörper führen, und daß diese dann bei der nachfolgenden, relativ unspezifischen Markierung nach einem Doppelantikörperverfahren oder durch markiertes Protein A markiert werden und falsche positive Ergebnisse vortäuschen.

Eher unter praktischen Gesichtspunkten, insbesondere im Hinblick auf den erforderlichen Arbeitsaufwand und die erzielbare Bestimmungsgenauigkeit, stellt es bei der Bestimmung von Autoantikörpern ein weiteres Problem dar, daß diese im Falle des Vorliegens einer Autoimmunkrankheit in außerordentlich hohen Mengen in den biologischen Flüssigkeiten, insbesonderen den Patientenseren, vorkommen. Bei einem Probenvolumen von 20 µl Serum ist z.B. mit bis zu 20 µg humanen Autoantikörpem zu rechnen. Um sie bestimmen zu können, insbesondere nach der "coated tube"-Technik oder Mikrotiterplatten-Technik, ist es daher normalerweise erforderlich, die Patientenseren mehrfach zu verdünnen, was arbeits- und zeitaufwendig ist und eine zusätzliche Fehlerquelle darstellt.

Angesichts der geschilderten Schwierigkeiten einer Bestimmung von Autoantikörpern nach den bekannten immundiagnostischen Bestimmungsverfahren besteht daher in dringender Bedarf nach einem neuartigen Verfahren zur immundiagnostischen Bestimmung von Autoantikörpern gegen hTPO in biologischen Flüssigkeiten, das eine sichere qualitative Bestimmung von Antikörpern in biologischen Flüssigkeiten ermöglicht, das durch geeignete Eichung und Optimierung der Verfahrensparameter auch zur zuverlässigen quantitativen Bestimmung von Antikörpern geeignet ist, bei dem unverdünnte Seren eingesetzt werden können und bei dem es nicht nötig ist, hochgereinigte Antigene zu den zu bestimmenden Antikörpem in nennenswerten Mengen einzusetzen.

Es ist Aufgabe der vorliegenden Erfindung, ein derartiges Verfahren zu schaffen. Diese Aufgabe wird durch ein immunologisches Bestimmungsverfahren gelöst, wie es im Patentanspruch 1 dargestellt wird.

Bevorzugte Ausführungsformen sind in den Unteransprüchen enthalten.

Bei dem erfindungsgemäßen Bestimmungsverfahren wird so vorgegangen, daß zum Nachweis der gesuchten Autoantikörper (Ak), die Störung der Ausbildung eines Sandwichkomplexes aus einem ersten immobilisierten Antikörper (Akᵢₘₘ), einem zugesetzten Antigen (Ag), insbesondere rohen Antigen, und einem weiteren Antikörper (Ak*), der eine nachweisbare Markierung trägt, festgestellt wird, die auf die Anwesenheit der zu bestimmenden Antikörper (Ak) in der biologischen Flüssigkeit zurückzuführen ist. Die Behinderung der Ausbildung eines Sandwichkomplexes der genannten Art äußerst sich in einer Verminderung der Bindung des markierten Antikörpers (Ak*) an die feste Phase. Die Störung der Ausbildung des Sandwich durch den in der Probe vorhandenen, zu bestimmenden Autoantikörper (Ak) kann dabei grundsätzlich an jeder einzelnen der Bindungsstellen zum Aufbau des Sandwich oder an beiden gleichzeitig erfolgen. Substanzen, die als Autoantigene wirken und gegen die Autoantikörper gebildet werden können, sind in der Regel Komplexe großer Moleküle, meist von proteinischer Natur, die mehr als zwei an der Antikörperbindung beteiligte Regionen oder Epitope aufweisen, und die gebildeten Antikörper sind polyklonal. Je nach Wahl der für den Test als immobilisierte bzw. markierte Antikörper ausgewählten Antikörper können daher Sandwichkomplexe verschiedener Art konstruiert werden, die sich - was die involvierten Bindungen und ihre Störungen angeht - gegenüber der Anwesenheit der Autoantikörper (Ak) ganz verschieden verhalten können. Die Konstruktion derartiger Sandwiches unter Vorgabe geeigneter Eigenschaften für den Nachweis von bestimmten Autoantikörpem (Ak) kann insbesondere dann maßgeschneidert werden, wenn sowohl der immobilisierte Antikörper (Akᵢₘₘ) als auch der markierte Antikörper (Ak*) geeignete monoklonale Antikörper sind. Im Falle der Bestimmung von Autoantikörpem gegenüber TPO sind die an diesem Antigen vorhandenen Epitope relativ gut charakterisiert, und es sind verschiedene monoklonale Antikörper verfügbar, die gegen spezifische Epitope dieses Antigens gerichtet sind. Es wird in diesem Zusammenhang verwiesen auf den bereits erwähnten Artikel von P. Carayon et al in Endocrinology, Volume 125, Nr. 3, Seiten 1211-1218.

Wählt man als immobilisierten Antikörper gegen TPO einen solchen, insbesondere monoklonalen, Antikörper, der TPO in einer Region bindet, in der auch Autoantikörper gegen TPO gebunden werden, und wählt man den markierten Antikörper Ak* so, daß seine Bindung durch Autoantikörper nicht beeinflußt wird, erfolgt die Störung des Aufbaus des Sandwiches aufgrund einer Konkurrenz zwischen dem immobilisierten Antikörper Akᵢₘₘ und dem zu bestimmenden Antikörper Ak um den Komplex Antigen-markierter Antikörper (Ag-Ak*), der als indirekt markiertes Antigen betrachtet werden kann. Geht man umgekehrt vor und wählt den immobilisierten Antikörper Akᵢₘₘ so, daß seine Bindung an das Antigen durch die zu bestimmenden Antikörper (Ak) nicht gestört wird, während der markierte Antikörper Ak* an Regionen oder Epitope des Antigens bindet, an die auch die zu bestimmenden Antikörper bzw. Autoantikörper binden, so erfolgt die Störung des Aufbaus eines Sandwiches, der markierten Antikörper Ak* enthält, aufgrund der Konkurrenz von Ak* und Ak um das indirekt immobilisierte Antigen. Binden beide Antikörper Akᵢₘₘ und Ak* an solche Bereiche des Antigens, an die auch die Autoantikörper (Ak) binden, kommt es durch die Anwesenheit derartiger Autoantikörper (Ak) zu einer doppelten Beeinträchtigung des Sandwichaufbaus und seiner Immobilisierung und damit gegebenenfalls zu einer Erhöhung der Empfindlichkeit.

Es ist ein sehr wesentlicher Vorteil des erfindungsgemäßen Verfahrens, daß das Antigen Ag nicht wie bei den bisher bekannten Bestimmungsverfahren in hochgereinigter Form vorliegen muß, sondern als rohes Antigen, beispielsweise in Form eines Organextrakts, verwendet werden kann. Die doppelte Antikörperspezifität, die zum Aufbau eines Sandwichs, der eine Markierung enthält, erforderlich ist, führt dazu, daß unspezifische Störungen des Tests durch Fremdbestandteile, die mit dem rohen Antigen in die Bestimmung eingeführt werden, ohne Schwierigkeiten eliminiert werden können, so daß die Sandwichbildung auf die gewünschten immunologischen Bindungspartner beschränkt ist. Während im oben beschriebenen Falle, bei dem man das rohe Antigen zusammen mit Begleitsubstanzen immobilisiert, die Gefahr besteht, daß nicht nur Antikörper gegen das immobilisierte Antigen an die feste Phase gebunden und markiert werden, sondern auch immunologische Bindungspartner zu anderen im rohen Antigen vorhandenen antigenen Substanzen, werden derartige Störungen bei dem erfindungsgemäßen Verfahren sehr unwahrscheinlich. Die Möglichkeit, ein rohes Antigen in Form eines geeigneten Organextrakts zu verwenden, hat den ferneren Vorteil, daß relativ schonende Gewinnungsverfahren für das Antigen angewandt werden können und auf solche Reinigungsschritte verzichtet werden kann, durch die die native Struktur des Antigens angegriffen wird. Das Antigen kann daher sehr viel genauer das im Organismus vorhandene Antigen und dessen vollständiges immunologisches Bindungsspektrum repräsentieren. Die Gefahr, daß nur eine Teilfraktion der im Organismus gebildeten Autoantikörper durch den Test erfaßt wird, ist somit geringer.

Andererseits läßt es sich durch eine geeignete Auswahl der für den Test verwendeten monoklonalen Antikörper auch erreichen, daß der Test ganz gezielt nur auf bestimmte Autoantikörper anspricht und damit selektiver gemacht werden kann. Wenn z.B. bestimmte mit der Autoimmunerkrankung verbundene Krankheitserscheinungen auf ganz bestimmte Klone der vom Körper gebildeten polyklonalen Autoantikörper zurückgeführt werden können, kann unter Verwendung monoklonaler oder ggf. selektierter polyklonaler Antikörper das Verfahren für die Bestimmung derartiger Autoantikörper-Klone spezifisch gemacht werden

Bei dem erfindungsgemäßen Verfahren stellen die zu erwartenden hohen Autoantikörperkonzentrationen kein Problem dar, da das rohe Antigen, d.h. rohe hTPO, in ausreichenden, den Autoantikörpermengen vergleichbaren Mengen eingesetzt werden kann, ohne den Test übermäßig zu verteuern. Es hat sich gezeigt, daß die Möglichkeit, das rohe Antigen zu verwenden, kostenmäßig so vorteilhaft ist, daß die bei dem erfindungsgemäßen Verfahren erforderlichen zwei zugesetzten Antikörper (Akᵢₘₘ, Ak*) keinen Kostennachteil gegenüber mit hochgereinigten Antigenen, jedoch nur einem Antikörper arbeitenden Verfahren bedeuten.

Das erfindungsgemäße Bestimmungsverfahren eignet sich ferner ausgezeichnet dazu, im Hinblick auf die gewünschte Empfindlichkeit optimiert zu werden, indem man die vorgesehenen Mengen an Antigen Ag bzw. markiertem Antikörper Ak* bzw immobilisiertem Antikörper Akᵢₘₘ an die Testbedingungen in weiten Grenzen anpaßt. Das ist aufgrund der guten Verfügbarkeit und des relativ geringen Preises des verwendeten rohen, nativen Antigens ohne größere Probleme möglich. Die Antigenmenge kann direkt auf die zu erwartenden oder möglichen Mengen an Autoantikörper abgestimmt werden, was gleichzeitig bedeutet, daß die biologischen Flüssigkeiten, d.h. insbesondere Seren, in unverdünnter Form eingesetzt werden können.

Was die einzusetzenden Mengen der immunologischen Umsetzungspartner angeht, so versteht es sich für den Fachmann von selbst, daß die Menge an zugesetztem Antigen nicht so groß sein sollte, daß sowohl alle zu bestimmenden Antikörper Ak als auch alle markierten Antikörper Ak* bzw. alle immobilisierten Antikörper Akᵢₘₘ abgesättigt werden und es zwischen ihnen zu keiner nennenswerten Konkurrenz um das Antigen mehr kommt. In einem solchen Falle kann eine nennenswerte Ausbildung eines immobilisierten, markierten Sandwiches u.U. überhaupt unterdrückt werden, und es sind keine quantitativen Aussagen über die tatsächlich in der Probe vorhandenen Autoantikörpermengen möglich.

Setzt man gegenüber der Menge des zugesetzten Antigens einen Unterschuß an z.B. markiertem Antikörper Ak* ein, so daß nur ein Teil der Antigenmenge unter Ausbildung eines Immunkomplexes Ag-Ak* markiert wird, darf natürlich die Menge des markierten Ak* nicht so gering sein, daß die schließlich gebundene Menge des Komplexes aufgrund seiner Konkurrenz mit unmarkiertem Antigen um den immobilisierten Antikörper Akᵢₘₘ zu niedrig wird. Analoges gilt, in einer anderen geschilderten Verfahrensvariante, für die Menge des immobilisierten Antikörpers Akᵢₘₘ.

Die für einen bestimmten Test erforderlichen oder vorteilhaftesten Konzentrationen an Antigen Ag, immobilisiertem Antikörper Akᵢₘₘ und markiertem Antikörper Ak* lassen sich unter Berücksichtigung der zu erwartenden Mengen der zu bestimmenden Antikörper Ak ohne nennenswerte Schwierigkeiten in Routineoptimierungen ermitteln, indem die Testempfindlichkeit (Eichkurvenverlauf) über z.B. die zugesetzten Antigenmengen variiert wird.

Antikörper, die bei dem erfindungsgemäßen Verfahren verwendet werden können, können im wesentlichen alle für immunologische Bestimmungsverfahren geeigneten Antikörper sein. Ihre Affinitätskonstanten liegen üblicherweise im Bereich von 10¹² bis 10⁸ l/mol.

Die Verfahrensdurchführung unterscheidet sich, was die verwendbaren festen Träger für die immobilisierten Antikörper Akᵢₘₘ und die Bedingungen der immunologischen Reaktion (pH zwischen 4 und 9, Anwesenheit von Puffern; Temperatur im Bereich von 0 bis ca. 55°C; Reaktionszeiten) angeht, nicht grundsätzlich von anderen üblichen immunologischen Bestimmungsverfahren. Die immunologische Umsetzung kann dabei unter solchen Bedingungen durchgeführt werden, daß das Gleichgewicht zwischen allen Reaktionspartnern erreicht wird, es ist jedoch grundsätzlich auch möglich, die Umsetzung zu einem vorgegebenen früheren Zeitpunkt zu stoppen und die Verhältnisse zu diesem früheren Zeitpunkt zu ermitteln.

Nachfolgend wird das erfindungsgemäße Verfahren anhand eines Ausführungsbeispiels, das die Bestimmung von Autoantikörpern gegen humane Schilddrüsenperoxidase (hTPO) unter Verwendung zweier zugesetzter monoklonaler Antikörper gegen hTPO sowie von roher hTPO in Form eines Extrakts aus humanen Schilddrüsen als zugesetztes Antigen betrifft, noch näher erläutert und hinsichtlich seiner Wirksamkeit bekannten Verfahren zur Bestimmung der gleichen Autoantikörper gegenübergestellt.

In den Figuren zeigen:

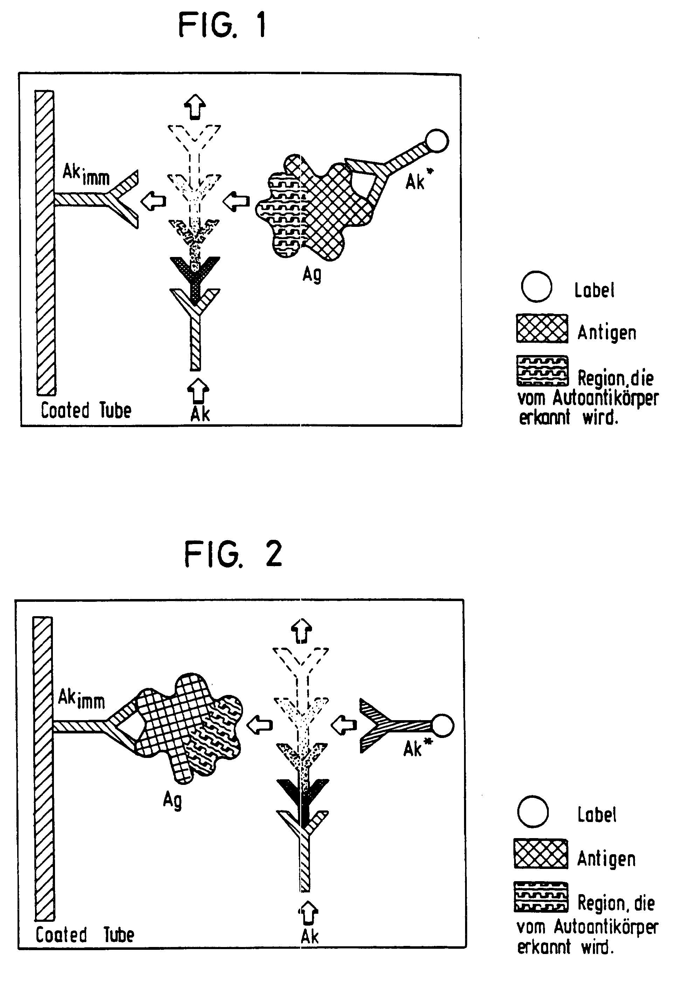

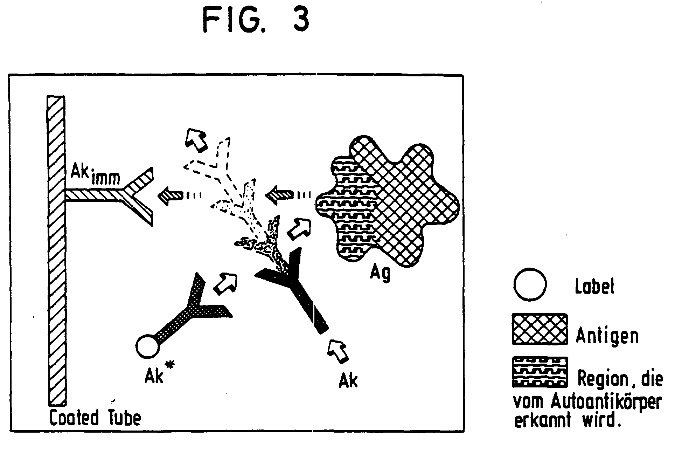

Figur 1 eine schematische Darstellung des erfindungsgemäßen Verfahrens in einer ersten Variante, in der der immobilisierte Antikörper Akᵢₘₘ an eine solche Region des Antigens Ag bindet, die auch von dem zu bestimmenden Antikörper Ak, der vorzugsweise ein Autoantikörper ist, erkannt wird, so daß der Antikörper Ak mit dem immobilisierten Antikörper Akᵢₘₘ um das Antigen Ag konkurriert und damit dessen Fixierung auf der festen Phase stört; die durch den Antikörper Ak hervorgerufene Störung ist hier und in den folgenden Figuren 2 und 3 symbolisch als intervenierende Bewegung des Antikörpers Ak dargestellt, obwohl, wie dem Fachmann ohne weiteres klar ist, die beobachtete Störung auf eine Konkurrenz um das Antigen zurückzuführen ist.
Figur 2 eine weitere Variante des erfindungsgemäßen Verfahrens, gemäß der der zu bestimmende Antikörper Ak und der markierte Antikörper Ak* um die Region des über Akᵢₘₘ indirekt immobilisierten Antigens Ag, die vom Autoantikörper Ak erkannt wird, konkurrieren.
Figur 3 eine dritte Variante des erfindungsgemäßen Verfahrens, gemäß der sowohl der immobilisierte Antikörper Akᵢₘₘ als auch der markierte Antikörper Ak* an eine Region des Antigens Ag binden, die auch von dem Antikörper Ak erkannt wird, so daß die Anwesenheit des Antikörpers Ak in der Probe eine doppelte Störung hervorruft;
Figur 4 eine graphische Darstellung des Standardkurvenverlaufs bei einem Assay zum Nachweis von humanen Autoantikörpem gegen humane Schilddrüsenperoxidase (hTPO) in Abhängigkeit von der eingesetzten Antigenmenge (rohes hTPO).

### Beispiel

Das nachfolgende Beispiel zeigt die Durchführung und die praktischen Vorteile eines Assays, der den Nachweis von humanen Autoantikörpern gegen humane Schilddrüsenperoxidase (hTPO) betrifft.

### 1. Immobilisierung eines monoklonalen anti-hTPO-Antikörpers (Akᵢₘₘ) auf einer festen Phase

Als Antikörper, der an die feste Phase gekoppelt wird, wurde ein gereinigter monoklonaler Antikörper gewählt, der an eine Region der hTPO bindet, die von humanen Autoantikörpern gegen hTPO erkannt wird. Der verwendete gereinigte monoklonale Antikörper war ein monoklonaler Maus-anti-hTPO-Antikörper, der hergestellt worden war gemäß dem in der Veröffentlichung von P. Carayon in: Endocrinology, Vol. 125, Nr. 3, Seite 1212, linke Spalte unten, rechte Spalte oben, beschriebenen Verfahren, und der nach den in der gleichen Veröffentlichung beschriebenen Auswahlkriterien so ausgewählt wurde, daß er hTPO in einer Region erkennt, die auch von humanen anti-hTPO-Autoantikörpern erkannt wird.

Die Kopplung des genannten monoklonalen Maus-anti-hTPO-Antikörpers an die feste Phase in Form der Wände eines Teströhrchens wurde nach bekannten Verfahren wie folgt durchgeführt:

Teströhrchen (STAR Tubes 12 x 75 mm der Firma NUNC, Katalog Nr. 470/319) wurden mit je 1 µg Anti-Maus-lgG (Firma SIGMA, Katalog Nr. MA 8642) in 300 µl einer wäßrigen Pufferlösung von pH 7,8 befüllt, die eine Konzentration von 10 mmol TRIS/HCL und 10 mmol NaCl aufwies. Nach einer 20stündigen Inkubation bei Raumtemperatur erfolgte ein zweimaliger Waschgang der Röhrchen. Abgesättigt wurden die Röhrchen mit einer Lösung aus 0,5 % BSA (bovines Serumalbumin; SIGMA, Katalog Nr. A 3294), d.h. die Röhrchen wurden mit der Absättigungslösung befüllt, 2 Stunden bei Raumtemperatur inkubiert, wonach der Inhalt dekantiert wurde. In einem nachfolgenden dritten Schritt erfolgte die Bindung des zugesetzten monoklonalen Maus-Anti-hTPO-Antikörpers an die feste Phase durch Immunextraktion aus einer Lösung des genannten monoklonalen Antikörpers, die diesen in einer Menge von 0,2 µg in 300 µl der oben genannten Pufferlösung enthielt, zu welchem Zwecke für 20 Stunden bei Raumtemperatur inkubiert wurde. Danach wurden die Röhrchen gewaschen, und es erfolgte eine letzte Absättigung mit der gleichen Absättigungslösung wie oben. Danach wurden die Röhrchen mit dem immobilisierten monoklonalen Antikörper lyophilisiert.

### 2. Herstellung eines hTPO-Extraktes aus humaner Schilddrüse

Gefrorene humane Schilddrüsen (60 g) wurden zerkleinert, mit Puffer (200 ml phosphatgepufferter Salzlösung, PBS) versetzt und anschließend mittels eines Homogenisators (Uitraturrax der Firma IKA Werke) homogenisiert. Nach einer einstündigen Zentrifugation bei 100 000 g wurde der Überstand entfernt, und das erhaltene Pellet wurde auf die gleiche Weise wie die zerkleinerten Schilddrüsen rehcmogenisiert. Daran schloß sich eine weitere Zentrifugation bei 100 000 g für 1 Stunde an. Das nunmehr erhaltene Pellet wurde wieder in PBS (200 ml) rehomogenisiert, das zusätzlich als Detergens 0,5 % Triton X 100 der Firma PIERCE (Katalog-Nr. 28314) enthielt, und es wurde 1 Stunde bei 4°C gerührt. Zuletzt wurde das erhaltene Homogenisat bei 100 000 g für 2 Stunden zentrifugiert. Der resultierende Überstand stellt den hTPO-Extrakt dar, der als rohes natives Antigen Ag in das erfindungsgemäße Verfahren zur Bestimmung von Autoantikörpern gegen hTPO eingesetzt wird.

### 3. Herstellung eines mit einem Chemilumineszenzlabel markierten Anti-hTPO-Antikörpers (Ak*)

Ein monoklonaler Maus-anti-hTPO-Antikörper, der grundsätzlich nach dem gleichen Verfahren erhalten wurde wie der oben unter 1. beschriebene entsprechende monoklonale Antikörper, der jedoch so ausgewählt wurde, daß er hTPO außerhalb der Region bindet, die auch von humanen Autoantikörpern gegen hTPO erkannt wird, wird nach bekannten Methoden mit Akridiniumester markiert. Zu diesem Zwecke wird der reine monoklonale Antikörper (100 µg in 100 µl PBS) mit Akridiniumester (2µg in 2 µl Acetonitril) für 10 min bei Raumtemperatur umgesetzt. Anschließend wird der mit dem Akridiniumester markierte Antikörper über HPLC von richt-umgesetztem freien Akridiniumester abgetrennt.

### 4. Bestimmung von humanen Autoantikörpern gegen hTPO

**a)** Zur Bestimmung von humanen Autoantikörpem gegen hTPO wurde im vorliegenden Falle zuerst der markierte Antikörper Ak* in einem molaren Verhältnis von 1:1 mit dem als Antigen Ag verwendeten hTPO-Extrakt umgesetzt. Die Umsetzung erfolgte innerhalb von 20 Stunden bei 4°C in einem Puffer, der folgende Bestandteile in den nachfolgend angegebenen Konzentrationen enthielt: 50 mmol Natriumphosphat, 0,1 Gew.-% Triton X 100, 0,2 Gew.-% EDTA, 0,3 % BSA (bovines Serumalbumin), 100 µg/ml Maus-lgG (Firma SIGMA, Nr. I 5381), 10 µg/ml bovines IgG (Firma SIGMA, Nr. 5506). Der pH des Puffers betrug 78.
**b)** Zum Nachweis von humanen Autoantikörpern gegen hTPO bzw. zur Eichung wurde jeweils wie folgt vorgegangen:
   1. Es wurden jeweils 20 µl der zu untersuchenden Probe bzw. des Standards oder Serums in Teströhrchen pipettiert, die gemäß dem unter 1. beschriebenen Verfahrensschritt mit Mausanti-hTPO-Antikörpem beschichtet worden waren.
   2. Anschließend wurden 300 µl des gemäß 4a) bereiteten Cocktails pipettiert, der das Antigen hTPO in Form des Extrakts aus humanen Schilddrüsen sowie den damit umgesetzten markierten Maus-anti-hTPO-Antikörper enthielt.
   3. Nach dem Abschluß der Zugabe wurde die erhaltenen Reaktionsmischung 3 Stunden bei Raumtemperatur unter Schütteln inkubiert. Danach wurden die Teströhrchen gewaschen, und die Menge des an die Wand des Teströhrchens gebundenen Akridiniumester-Tracers wurde in einem Berthold-Autoclinilumaten LB 952/16 auf an sich bekannte Weise mittels der Lichtreaktion gemessen.

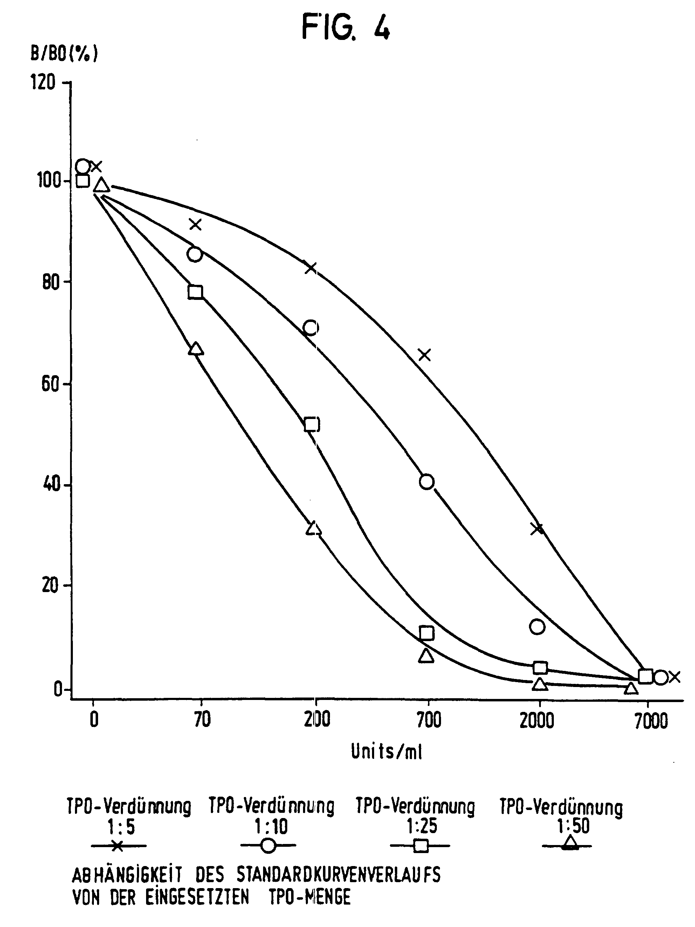

Figur 4 zeigt die für verschiedene Mengen von Autoantikörpem in der zu untersuchenden Probe bzw. dem verwendeten Standard erhaltenen Meßkurven für Assays mit unterschiedlichen Mengen an zugesetztem Antigen (hTPO) in Form eines humanen Schilddrüsenextrakts, wobei die Angabe "TPO-Verdünnung" sich auf den oben unter 2. beschriebenen Schilddrüsenmembranextrakt bezieht. Figur 4 ist klar zu entnehmen, daß über eine Variation der eingesetzten Antigen-Menge (hTPO-Menge) der Kurvenverlauf und darnit die Meßempfindlichkeit variiert werden kann.

### 5. Klinische Daten

In einer klinischen Untersuchung wurden die mit dem beschriebenen neuartigen Verfahren erhaltenen Meßergebnisse mit denen verglichen, die unter Verwendung existierender immunologischer Bestimmungsverfahren zur Autoantikörperbestimmung gegen hTPO für 29 positiv reagierende Patienten erhalten werden.

Die Ergebnisse sind in Tabelle 1 zusammengefaßt.

**Tabelle 1**

| Autoantikörper Units/ml | | | | |
|---|---|---|---|---|
| | a)* | b)* | c)* | d) Neues Verfahren |
| Normkollektiv Patienten-Poole (N=25) | alle neg. | alle neg. | alle neg. | alle neg. |
| Patient-Nr. | | | | |
| 1 | 156 | neg. | 168 | 138 |
| 2 | neg. | neg. | neg. | neg. |
| 3 | 129 | 83 | 240 | 207 |
| 4 | 3869 | 1163 | 8706 | 3742 |
| 5 | 2225 | 1414 | 2163 | 2235 |
| 6 | 182 | 91 | 249 | 147 |
| 7 | 198 | 598 | 2596 | 1728 |
| 8 | 3232 | 1006 | 5283 | 4834 |
| 9 | 1015 | 110 | 362 | 784 |
| 10 | neg. | neg. | 110 | neg. |
| 11 | 433 | 198 | 886 | 600 |
| 12 | 1049 | 359 | 1171 | 1473 |
| 13 | 140 | 89 | 168 | 135 |
| 14 | neg. | neg. | neg. | neg. |
| 15 | 134 | 86 | 255 | 94 |
| 16 | 2768 | 7496 | 5106 | 5159 |
| 17 | 2154 | 1412 | 4090 | 2791 |
| 18 | 341 | 105 | neg. | 109 |
| 19 | 898 | 530 | 1173 | 1552 |
| 20 | 1478 | 476 | 1125 | 1471 |
| 21 | 3753 | 2215 | 2593 | 5069 |
| 22 | 274 | 108 | 141 | 237 |
| 23 | 784 | 516 | 461 | 414 |
| 24 | 921 | 472 | 521 | 451 |
| 25 | 405 | 341 | 798 | 709 |
| 26 | 1415 | 424 | 997 | 1032 |
| 27 | 1914 | 1011 | 2608 | 2170 |
| 28 | 183 | 704 | 2631 | 1747 |
| 29 | 161 | neg. | 130 | 378 |

| | | | | |
|---|---|---|---|---|
| * : Ergebnisse von Vergleichsverfahren | | | | |

In dieser Tabelle 1 betreffen die Spalten a), b) und c) die Ergebnisse von Bestimmungsverfahren des Standes der Technik, die nachfolgend noch genauer erläutert werden, während d) die nach dem neuen, wie vorstehend beschrieben durchgeführten Verfahren erhaltenen Ergebnisse zeigt.

Die als Vergleichsverfahren dem erfindungsgemäßen Verfahren gegenübergestellten Verfahren des Standes der Technik waren im einzelnen:
a) Ein Verfahren, bei dem ein Anti-hTPO-Antikörper auf der Festphase verwendet wird. Radioaktiv markiertes und gereinigtes hTPO wird durch humane Autoantikörper von der Festphase verdrängt. Der verwendete Test ist ein kommerziell erhältlicher Test, der als DYNOtest Anti-TPO der Anmelderin im Handel ist.
b) Bei diesem Test ist Protein A auf der Festphase immobilisiert. Der Nachweis von Autoantikörpern in einer Probe erfolgt durch deren Bindung an die Festphase und ihren anschließenden Nachweis mit Hilfe von markiertem gereinigtem hTPO. Im konkreten Falle wird radiojodiertes hTPO gemäß dem IMMUtest Anti-TPO der Anmelderin verwendet.
c) Bei diesem Test wird rohes TPO (als sogenanntes mikrosomales Antigen) auf der Festphase immobilisiert eingesetzt. Die Bindung und der Nachweis der gebundenen Autoantikörper erfolgt mit Hilfe eines markierten Protein A. Bei dem Vergleichstest handelt es sich um den PROMAK-Assay der Anmelderin.

Der Tabelle 1 mit den Vergleichsergebnissen ist deutlich zu entnehmen, daß das Vergleichsverfahren a) (gereinigtes markiertes hTPO als Tracer) sowie das Vergleichsverfahren b) (Protein A auf einer Festphase, gereinigtes markiertes hTPO als Tracer) teilweise niedrigere Werte liefem als das Bestimmungsverfahren gemäß c) und das erfindungsgemäße Verfahren d). So werden gemäß Bestimmungsverfahren a) für einzelne Patientenproben (Patienten 7, 16, 28) deutlich niedrigere Werte erhalten als nach den restlichen Verfahren, während gemäß Bestimmungsverfahren b) für einige Patienten negative Ergebnisse erhalten werden, die nach den anderen Verfahren schwach positiv sind (Patienten 1, 29) bzw. für einige Patienten niedrigere Werte ermittelt werden als nach den anderen Bestimmungsverfahren (Patienten 4, 8, 11, 12, 17, 19, 20, 27). Es ist darauf hinzuweisen, daß im Falle der Patienten 1 und 29 die Proben gemäß Bestimmungsverfahren b) sogar unrichtig als autoantikörperfrei ermittelt wurden. Im Falle der Patienten 7 und 28 findet das Bestimmungsverfahren a) die Patienten nur als extrem schwach positiv, während sie in den anderen Verfahren stark positiv sind.

Verfahren c) hat den Nachteil, daß neben Antikörpern gegen hTPO auch eine Vielzahl weiterer Autoantikörper gegen begleitende Proteine gemessen werden, was beispielsweise dazu führt, daß für den Patienten 10, der gemäß allen anderen Verfahren frei von Autoantikörpem gegen hTPO ist, Autoantikörper gegen hTPO vorgetäuscht werden.

Das dem erfindungsgemäßen Verfahren zugrundeliegende Verfahrensprinzip ist - wie eingangs ausführlich erläutert - in vielfacher Hinsicht variierbar. Da ein Antikörper markiert ist, kann jedes z.Zt. bekannte Label zur Markierung eingesetzt werden. Anstelle der im konkreten Falle geschilderten vorausgehenden Umsetzung von Antigen (rohem hTPO) mit dem markierten Antikörper ist es auch möglich, das Verfahren als Synchronreaktion durchzuführen, d.h. das rohe Antigen (hTPO) wird nicht vor der Zugabe der zu untersuchenden Probe in einem getrennten Schritt mit dem markierten Antikörper indirekt vormarkiert, sondern die Inkubation wird folgendermaßen durchgeführt:

In das mit dem immobilisierten Antikörper beschichtete Teströhrchen wird zuerst die auf Autoantikörper zu untersuchende Probe pipettiert, anschließend wird der markierte Antikörper Ak* zugegeben, und als letztes wird die Antigenlösung (hTPO-Lösung) zugegeben, womit die Reaktion gestartet wird.

Ein solches Vorgehen ist dann von Vorteil, wenn die Bindungsverhältnisse so sind, daß humane Autoantikörper auch die Wechselwirkung zwischen dem markierten Antikörper Ak* und dem Antigen Ag (hTPO) beeinflussen und schwächen, und zwar etwa im Sinne des Verfahrensschemas gemäß Figur 3.

Andere Pipettierschemate sind ebenfalls möglich. Beispielsweise ist es auch möglich, die Immobilisierung des ersten Antikörpers Akᵢₘₘ erst während des Bestimmungsverfahrens vorzunehmen, indem man im Testgefäß eine Festphase mit einem Binder für Akᵢₘₘ vorlegt und anschließend z.B. eine Mischung aus Akᵢₘₘ und Ak* pipettiert und zuletzt durch Zugabe von Antigen Ag die Umsetzung startet.

Auch wenn der Wegfall des Zwangs zur Vorverdünnung der Proben einen wesentlichen Vorteil des erfindungsgemäßen Verfahrens darstellt, ist es bei Bedarf selbstverständlich auch möglich, den Test so zu variieren, daß die Proben vorverdünnt eingesetzt werden.

## Patentansprüche

1. Immunologisches Bestimmungsverfahren zur Bestimmung von Autoantikörpern (Ak) gegen Schilddrüsenperoxidase (TPO) in biologischen Flüssigkeiten, rach einem Verfahren, bei dem die biologische Flüssigkeit umgesetzt wird mit
a) einer vorgegebenen Menge des Antigens (Ag) zu den zu bestimmenden Antikörpern (Ak), und
b) einem Antikörper (Akᵢₘₘ), der für eine bestimmte, an der Antikörperbindung beteiligte Region R¹ des Antigens (Ag) spezifisch ist, in einer durch Bindung an eine feste Phase oder Mikrofestphase immobilisierten Form,
und bei dem man anschließend die feste Phase von der flüssigen Phase trennt und die Menge einer über das Antigen (Ag) an die feste Phase gebundenen oder in der flüssigen Phase verbliebenen Markierung feststellt und dann durch qualitative Bewertung oder durch rechnerische Auswertung der erhaltenen Meßergebnisse unter Verwendung von Eichkurven die Anwesenheit bzw. die Menge der zu bestimmenden Autoantikörper in der biologischen Probe ermittelt,
**dadurch gekennzeichnet,** daß als Antigen (Ag) rohe native Schilddrüsenperoxidase (TPO) in unmarkierter Form eingesetzt wird, und daß bei der Bestimmung ein weiterer Antikörper (Ak*) zugegen ist, der einen detektierbaren Markierungsanteil aufweist und ebenfalls an das TPO-Antigen (Ag) bindet, und daß die Anwesenheit und/oder Menge der zu bestimmenden Autoantikörper (Ak) gegen Schilddrüsenperoxidase (TPO) in der zu untersuchenden biologischen Flüssigkeit ermittelt wird anhand der Verminderung des an die feste Phase gebundenen Anteils des markierten Antikörpers (Ak*), verglichen mit der Menge des an die feste Phase gebundenen bzw. in der flüssigen Phase verbliebenen markierten Antikörpers (Ak*) bei Verwendung einer von den zu bestimmenden Autoantikörpern (Ak) gegen Schilddrüsenperoxidase freien Probe der biologischen Flüssigkeit oder einer Standard-Blindprobe.

2. Bestimmungsverfahren nach Anspruch 1, **dadurch gekennzeichnet**, daß der immobilisierte Antikörper (Akᵢₘₘ) und/oder der markierte Antikörper (Ak*) monoklonale Antikörper sind.

3. Bestimmungsverfahren nach Anspruch 1 oder 2, **dadurch gekennzeichnet**, daß der markierte Antikörper (Ak*) entweder
a) an eine andere, die Bindung des nativen TPO-Antigens (Ag) an den immobilisierten Antikörper (Akᵢₘₘ) nicht beeinflussende Region R² des zugesetzten nativen TPO-Antigens bindet, oder
b) an eine solche Region R³ des zugesetzten nativen TPO-Antigens (Ag) bindet, daß seine Bindung an das native TPO-Antigen (Ag) unter Ausbildung eines Komplexes (Ag-Ak*) durch
b₁) den immobilisierten Antikörper und/oder
b₂) den zu bestimmenden Autoantikörper (Ak) gegen Schilddrüsenperoxidse vermindert wird und/oder seine Bindung ihrerseits die Bindung des immobilisierten Antikörpers (Akᵢₘₘ) an das native TPO-Antigen (Ag) vermindert.

4. Bestimmungsverfahren nach Anspruch 1, **dadurch gekennzeichnet**, daß die als Antigen (Ag) verwendete rohe native Schilddrüsenperoxidase (TPO) in Form eines Extrakts aus zerkleinerten humanen Schilddrüsen eingesetzt wird.

5. Bestimmungsverfahren nach einem der Ansprüche 1 bis 4, **dadurch gekennzeichnet**, daß der markierte Antikörper (Ak*) ein monoklonaler Antikörper ist, der an eine andere an der Antikörperbindung beteiligte Region des nativen TPO-Antigens (Ag) bindet als der immobilisierte monoklonale Antikörper (Akᵢₘₘ), und daß er und/oder das native TPO-Antigen in einer solchen Menge verwendet werden, daß es zu einer Konkurrenz des immobilisierten Antikörpers (Ak*) und der zu bestimmenden Autoantikörper (Ak) gegen Schilddrüsenperoxidase um die Komplexe aus nativem TPO-Antigen und markiertem Antikörper (Ag-Ak*) kommt.

6. Bestimmungsverfahren nach einem der Ansprüche 1 bis 4, **dadurch gekennzeichnet**, daß der markierte Antikörper (Ak*) ein monoklonaler Antikörper ist, der an eine solche an der Antikörperbindung beteiligte Region des nativen TPO-Antigens (Ag) bindet, daß die Ausbildung des Komplexes aus nativem TPO-Antigen (Ag) und markiertem Antikörper (Ag-Ak*) sowie gegebenenfalls zusätzlich die Bindung dieses Komplexes (Ag-Ak*) an den an der festen Phase immobilisierten Antikörper (Akᵢₘₘ) bei Anwesenheit der zu bestimmenden Autoantikörper (Ak) gegen Schilddrüsenperoxidase in der Bestimmungsmischung vermindert wird.

7. Bestimmungsverfahren nach einem der Ansprüche 1 bis 6, **dadurch gekennzeichnet**, daß die auf die Anwesenheit der Autoantikörper (Ak) gegen Schilddrüsenperoxidase zu untersuchende biologische Flüssigkeit, insbesondere in Form eines humanen Serums, in unverdünnter Form in das Bestimmungsverfahren eingesetzt wird.

8. Bestimmungsverfahren nach einem der Ansprüche 1 bis 7, **dadurch gekennzeichnet**, daß der an den markierten Antikörper (Ak*) gebundene detektierbare Markierungsanteil ein radioaktives Isotop, ein Enzym, ein Fluoreszenz- oder Chemilumineszenzlabel oder ein Substrat für eine enzymatische Nachweisreaktion oder ein anderes bekanntes, bei immunologischen Bestimmungsverfahren verwendetes Label ist.

9. Bestimmungsverfahren nach einem der Ansprüche 1 bis 8, **dadurch gekennzeichnet**, daß die einzelnen, an der Bestimmung beteiligten Partner (Akᵢₘₘ, Ag, Ak*, Probe) zur Herstellung der Bestimmungsmischung in einer der folgenden Reihenfolgen pipettiert werden:
- in ein Testgefäß, das eine Festphase mit (Akᵢₘₘ) enthält, werden nacheinander a) die zu untersuchende Probe und b) der markierte Antikörper (Ak*) und das native TPO-Antigen (Ag) entweder einzeln nacheinander oder als ein sowohl (Ak*) und (Ag) enthaltender Cocktail pipettiert; oder
- in ein Testgefäß, das eine Festphase mit einem Binder für die Immobilisierung von (Akᵢₘₘ) enthält, werden nacheinander a) die Probe, b) eine Mischung der Antikörper (Akᵢₘₘ) und (Ak*) und c) das native TPO-Antigen (Ag) pipettiert.

10. Kit zur Durchführung eines Bestimmungsverfahrens nach einem der Ansprüche 1 bis 9, **dadurch gekennzeichnet**, daß er mindestens die folgenden Bestandteile enthält:
eine Festphase, an die der Antikörper (Akᵢₘₘ) immobilisiert ist oder während des Bestimmungsverfahrens immobilisiert wird; eine den markierten Antikörper (Ak*) enthaltende Lösung und
eine ein natives TPO-Antigen (Ag) enthaltende Lösung
oder eine sowohl den markierten Antikörper (Ak*) als auch das native TPO-Antigen (Ag) enthaltende Lösung.

11. Kit nach Anspruch 10, **dadurch gekennzeichnet**, daß er als natives TPO-Antigen (Ag) rohe native TPO in Form eines Extrakts aus zerkleinerten humanen Schilddrüsen enthält.

## Claims

1. Immunological assay method for the determination of autoantibodies (Ak) against thyroid peroxidase (TPO) in biological fluids, by applying a method in which the biological fluid is reacted with
a) a predetermined amount of the antigen (Ag) against the antibodies (Ak) to be determined and
b) an antibody (Akᵢₘₘ) which is specific for a certain region R¹ of the antigen (Ag) which is involved in antibody binding, in a form in which it is immobilised by binding to a solid phase or microsolid phase,
and in which the solid phase is then separated from the liquid phase and the amount of a label bound via the antigen (Ag) to the solid phase, or remaining in the liquid phase, is then determined and the presence or the amount of the autoantibodies to be determined in the biological sample is then determined by qualitative assessment or by computational evaluation of the results obtained, using calibration curves,
**characterised in that** as antigen (Ag) crude native thyroid peroxidase (TPO) in unlabelled form is used, and that a further antibody (Ak*) which has a detectable label part and which likewise binds to the TPO-antigen (Ag) is present during the assay, and that the presence and/or amount of the autoantibodies (Ak) against thyroid peroxidase (TPO) to be determined in the biological fluid to be investigated is determined on the basis of the reduction of the proportion of the labelled antibody (Ak*) which is bound to the solid phase, compared with the amount of the labelled antibody (Ak*) bound to the solid phase, or remaining in the liquid phase, if a sample of the biological fluid which is free from the autoantibodies (Ak) against thyroid peroxidase to be determined, or a standard blank sample, is used.

2. Assay method according to Claim 1, **characterised in that** the immobilised antibody (Akᵢₘₘ) and/or the labelled antibody (Ak*) are monoclonal antibodies.

3. Assay method according to Claim 1 or 2, **characterised in that** the labelled antibody (Ak*) either
a) binds to another region R² of the added native TPO-antigen (Ag), which region does not affect the binding of the native TPO-antigen (Ag) to the immobilised antibody (Akᵢₘₘ), or
b) binds to a region R³ of the added native TPO-antigen (Ag) such that its binding to the native TPO-antigen (Ag) and the formation of a complex (Ag-Ak*) is reduced by
b₁) the immobilised antibody and/or
b₂) the autoantibody (Ak) against thyroid peroxidase to be determined, and/or its binding in turn reduces the binding of the immobilised antibody (Akᵢₘₘ) to the native TPO-antigen (Ag).

4. Assay method according to Claim 1, **characterised in that** the crude native thyroid peroxidase (TPO) used as the antigen (Ag) is employed in the form of an extract of comminuted human thyroids.

5. Assay method according to any of Claims 1 to 4, **characterised in that** the labelled antibody (Ak*) is a monoclonal antibody which binds to a region of the native TPO-antigen (Ag) which is involved in antibody binding but differs from the region to which the immobilised monoclonal antibody (Akᵢₘₘ) binds, and that it and/or the native TPO-antigen are used in an amount such that the immobilised antibody (Ak*) and the autoantibodies (Ak) against thyroid peroxidase to be determined compete for the complexes of native TPO-antigen and labelled antibody (Ag-Ak*).

6. Assay method according to any of Claims 1 to 4, **characterised in that** the labelled antibody (Ak*) is a monoclonal antibody which binds to a region of the native TPO-antigen (Ag) involved in antibody binding such that the formation of the complex of native TPO-antigen (Ag) and labelled antibody (Ag-Ak*), and possibly in addition the binding of this complex (Ag-Ak*) to the antibody (Akᵢₘₘ) immobilised on the solid phase, are reduced when the autoantibodies (Ak) against thyroid peroxidase to be determined are present in the assay mixture.

7. Assay method according to any of Claims 1 to 6, **characterised in that** the biological fluid, in particular in the form of a human serum, to be investigated for the presence of autoantibodies (Ak) against thyroid peroxidase is used in undiluted form in the assay method.

8. Assay method according to any of Claims 1 to 7, **characterised in that** the detectable label part bound to the labelled antibody (Ak*) is a radioactive isotope, an enzyme, a fluorescent or chemiluminescent label or a substrate for an enzymatic detection reaction, or another known label as used in immunological assay methods.

9. Assay method according to any of Claims 1 to 8, **characterised in that** the individual partners (Ak_{imm,} Ag, Ak*, sample) involved in the determination are pipetted in one of the following orders for the preparation of the assay mixture:
- a) the sample to be investigated and b) the labelled antibody (Ak*) and the native TPO-antigen (Ag), either individually in succession or as a cocktail containing both (Ak*) and (Ag), are pipetted in succession into a test vessel which contains a solid phase comprising (Akᵢₘₘ) ; or
- a) the sample, b) a mixture of the antibodies (Akᵢₘₘ) and (Ak*) and c) the native TPO-antigen (Ag) are pipetted in succession into a test vessel which contains the solid phase comprising a binder for immobilising (Akᵢₘₘ).

10. Kit for carrying out an assay method according to any of Claims 1 to 9, **characterised in that** it contains at least the following components:
a solid phase on which the antibody (Akᵢₘₘ) has been immobilised or is immobilised during the assay procedure;
a solution containing the labelled antibody (Ak*) and
a solution containing the native TPO-antigen (Ag);
or a solution containing both the labelled antibody (Ak*) and the native TPO-antigen (Ag).

11. Kit according to Claim 10, **characterised in that** it contains, as the native TPO-antigen, a crude native TPO in the form of an extract of comminuted human thyroids.

## Revendications

1. Procédé de détermination immunologique, pour la détermination d'auto-anticorps (Ak) dirigés vers la peroxydase de la glande thyroïde (TPO), dans des fluides biologiques, par application d'un procédé dans lequel le fluide biologique est mis à réagir avec
a) une quantité prédéterminée de l'antigène (Ag) dirigé vers les anticorps (Ak) à déterminer, et
b) un anticorps (Akᵢₘₘ) qui est spécifique pour une région R¹ définie de l'antigène (Ag), impliquée dans la liaison des anticorps, sous une forme immobilisée par liaison à une phase solide ou à une phase microsolide, et dans lequel on sépare ensuite la phase solide de la phase liquide, et l'on détermine la quantité d'un marqueur lié par l'intermédiaire de l'antigène (Ag) à la phase solide, ou restant dans la phase liquide, et l'on détermine ensuite la présence ou la quantité des auto-anticorps à déterminer dans l'échantillon biologique par évaluation qualitative ou par interprétation par calcul des résultats de mesure obtenus, en utilisant des courbes d'étalonnage, **caractérisé en ce que**, comme antigène (Ag), on utilise de la peroxydase de la glande thyroïde (TPO) native, brute, sous une forme non marquée, et en ce qu'un anticorps supplémentaire (Ak*) qui présente une fraction marquée détectable et qui se lie également à l'antigène TPO (Ag) est présent pendant le test, et en ce que la présence et/ou la quantité des auto-anticorps (Ak) dirigés vers la peroxydase de la glande thyroïde (TPO) à déterminer dans le fluide biologique à étudier, est déterminée sur base de la diminution de la fraction de l'anticorps marqué (Ak*) lié à la phase solide, comparée à la quantité de l'anticorps marqué (Ak*) lié à la phase solide, ou restant dans la phase liquide, lorsque l'on utilise un échantillon du fluide biologique exempt des auto-anticorps (Ak) dirigés vers la peroxydase de la glande thyroïde à déterminer, ou un échantillon à blanc standard.

2. Procédé de détermination selon la revendication 1, **caractérisé en ce que** l'anticorps immobilisé (Akᵢₘₘ) et/ou l'anticorps marqué (Ak*) sont des anticorps monoclonaux.

3. Procédé de détermination selon la revendication 1 ou 2, **caractérisé en ce que** l'anticorps marqué (Ak*)
a) soit se lie à une autre région R² de l'antigène TPO natif (Ag) ajouté, laquelle région n'influence pas la liaison de l'antigène TPO natif à l'anticorps immobilisé (Akᵢₘₘ),
b) soit se lie à une région R³ de l'antigène TPO natif (Ag) ajouté, telle que sa liaison a l'antigène TPO natif (Ag) avec formation d'un complexe (Ag-Ak*), est diminuée par
b₁) l'anticorps immobilisé et/ou
b₂) l'auto-anticorps (Ak) à déterminer, dirigé vers la peroxydase de la glande thyroïde, et/ou que sa liaison réduit à son tour la liaison de l'anticorps immobilisé (Akᵢₘₘ) à l'antigène TPO natif (Ag).

4. Procédé de détermination selon la revendication 1, **caractérisé en ce que** la peroxydase de la glande thyroïde (TPO) native, brute, utilisée comme antigène (Ag) est utilisée sous la forme d'un extrait de glande thyroïde humaine broyée.

5. Procédé de détermination selon l'une des revendications 1 à 4, **caractérisé en ce que** l'anticorps marqué (Ak*) est un anticorps monoclonal qui se lie à une région de l'antigène TPO natif (Ag), impliquée dans la liaison des anticorps, autre que celle à laquelle se lie l'anticorps monoclonal immobilisé (Akᵢₘₘ), et en ce que lui, et/ou l'antigène TPO natif, sont utilisés en une quantité telle qu'il se produit une concurrence entre l'anticorps immobilisé (Ak*) et l'auto-anticorps (Ak) dirigé vers la peroxydase de la glande thyroïde, à déterminer, et le complexe constitué de l'antigène TPO natif et de l'anticorps marqué (Ag-Ak*).

6. Procédé de détermination selon l'une des revendications 1 à 4, **caractérisé en ce que** l'anticorps marqué (Ak*) est un anticorps monoclonal qui se lie à une région de l'antigène TPO natif (Ag) impliquée dans la liaison aux anticorps, telle que la formation du complexe constitué de l'antigène TPO natif (Ag) et de l'anticorps marqué (Ag-Ak*) ainsi qu'éventuellement également la liaison de ce complexe (Ag-Ak*) avec l'anticorps (Akᵢₘₘ) immobilisé dans la phase solide, est diminuée en cas de présence de l'auto-anticorps (Ak) dirigé vers la peroxydase de la glande thgyroïde, à déterminer, dans le mélange de détermination.

7. Procédé de détermination selon l'une des revendications 1 à 6, **caractérisé en ce que** le fluide biologique, présentant en particulier la forme d'un sérum humain, dans lequel on étudie la présence de l'auto-anticorps (Ak) dirigé vers la peroxydase de la glande thyroïde, est utilisé sous une forme non diluée dans le procédé de détermination.

8. Procédé de détermination selon l'une des revendications 1 à 7, **caractérisé en ce que** la fraction de marquage détectable liée à l'anticorps marqué (Ak*) est un isotope radioactif, une enzyme, un traceur fluorescent ou chimiluminescent ou un substrat pour une réaction enzymatique de dépistage ou un autre traceur connu utilisé dans des procédés de détermination immunologiques.

9. Procédé de détermination selon l'une des revendications 1 à 8, **caractérisé en ce que** les partenaires individuels concernés par la détermination (Akᵢₘₘ, Ag, Ak*, échantillon) sont pipettés pour la préparation du mélange de détermination selon. l'une des séquences suivantes:
- dans un récipient d'essai, qui contient une phase solide comportant (Akᵢₘₘ), on pipette successivement a) l'échantillon à étudier et b) l'anticorps (Ak*) marqué et l'antigène TPO natif (Ag), soit individuellement l'un après l'autre soit sous la forme d'un cocktail contenant aussi bien (Ak*) que (Ag); ou
- dans un récipient d'essai qui contient une phase solide comportant un liant pour l'immobilisation de (Akᵢₘₘ), on pipette successivement a) l'échantillon, b) un mélange des anticorps (Akᵢₘₘ) et (Ak*) et c) l'antigène TPO natif (Ag).

10. Kit en vue de la mise en oeuvre d'un procédé de détermination selon l'une des revendications 1 à 9, **caractérisé en ce qu**'il contient au moins les composants suivants:
une phase solide sur laquelle l'anticorps (Akᵢₘₘ) est immobilisé, ou sur laquelle il est immobilisé au cours du procédé de détermination; une solution contenant l'anticorps marqué (Ak*) et une solution contenant l'antigène TPO natif (Ag), ou une solution contenant à la fois l'auto-anticorps marque (Ak*) et l'antigène TPO natif (Ag).

11. Kit selon la revendication 10, **caractérisé en ce qu**'il contient comme antigène TPO natif, une TPO native brute sous la forme d'un extrait de glande thyroïde humaine broyée.
